# EUROPEAN PATENT SPECIFICATION

(11) **EP 1 480 667 B1**
(45) Date of publication and mention of the grant of the patent: **06.12.2006**
(21) Application number: 03743340.6
(22) Date of filing: 26.02.2003
(51) Int. Cl.: A61K 38/02, A61K 35/42, A61P 11/00

(54) **COMPOSITIONS COMPRISING PULMONARY SURFACTANTS AND A POLYMYXIN HAVING IMPROVED SURFACE PROPERTIES**
ZUSAMMENSETZUNGEN MIT LUNGEN-SURFACTANTS UND POLYMYXIN MIT VERBESSERTEN OBERFLÄCHENEIGENSCHAFTEN
COMPOSITIONS CONTENANT DES TENSIOACTIFS PULMONAIRES ET UNE POLYMYXINE AUX PROPRIETES DE SURFACE AMELIOREES

(30) Priority: 01.03.2002 EP 02004789
(43) Date of publication of application: 01.12.2004
(73) Proprietor: CHIESI FARMACEUTICI S.p.A., I-43100 Parma (IT)
(72) Inventor: JOHANSSON, Jan, I-43100 Parma (IT); CURSTEDT, Tore, I-43100 Parma (IT); ROBERTSON, Bengt, I-43100 Parma (IT)
(74) Representative: Minoja, Fabrizio
(86) International application number: PCT/EP2003/001963
(87) International publication number: WO 2003/074070

(56) References cited:
- WO-A-00/47623
- ZALTASH^A S ET AL: "Pulmonary surfactant protein B: a structural model and a functional analogue" BIOCHIMICA ET BIOPHYSICA ACTA. BIOMEMBRANES, AMSTERDAM, NL, vol. 1466, no. 1-2, 1 June 2000 (2000-06-01), pages 179-186, XP004273236 ISSN: 0005-2736

## Description

This invention is directed to pulmonary surfactants comprising additives for improving their surface tension lowering properties.

Furthermore the invention is directed to the use of polymyxin for improving the properties of modified natural surfactants or reconstituted/artificial surfactants.

More particularly this invention is directed to the use of polymyxins for increasing the resistance to inactivation of modified natural surfactants or artificial surfactants and/or increasing their activity.

The modified natural surfactants or synthetic surfactants fortified with the polymyxins can be advantageously employed for the treatment of various lung diseases such as adult and neonatal respiratory distress syndromes, meconium aspiration syndrome, pneumonia and chronic lung disease.

### Background of the invention

Lung injury is a major clinical problem that includes diseases such as acute respiratory distress syndrome in adults (ARDS), neonatal respiratory distress syndrome (RDS), meconium aspiration syndrome (MAS), several types of pneumonia and bronchopulmonary dysplasia (BPD).

A common underlying event of all these diseases seems to be pulmonary surfactant deficiency and/or dysfunction.

Pulmonary surfactant is a lipid-protein mixture that coats the inside of the alveoli. The presence of the lipids as a monolayer at the air-liquid interface in the alveoli reduces the surface tension. Thereby it diminishes the tendency of alveoli to collapse during expiration and perhaps also reduces the transudation of fluid into the air spaces.

Endogenous pulmonary surfactant contains about 80 weight % phospholipids, 10 weight % neutral lipids, and 10 weight % proteins. Four surfactant proteins (SP) have been characterised, namely SP-A, SP-B, SP-C and SP-D (Johansson J et al. *Eur J Biochem* 1997, 244, 675-693).

Pulmonary surfactant deficiency and/or dysfunction can be either primary like in RDS or secondary like in ARDS, MAS and BPD (Robertson B Monaldi Arch Chest Dis 1988, 53, 64-69).

In ARDS and MAS, surfactant insufficiency or surfactant inactivation follows, for example, leakage of proteins, e.g. albumin or other surfactant inhibitors into the alveoli.

Also the resistance against pneumonia, which is an important cause of respiratory failure, is greatly reduced by the lack of surfactant, as surfactant plays an important role in the lung's defense against infection (Walther FJ in *Surfactant Therapy for Lung Disease,* Robertson & Taeusch, Eds.; Dekker, 1995, pp. 461-476).

Replacement therapy with a variety of exogenous surfactant has proved beneficial in both experimental and clinical studies.

According to Wilson (Expert Opin Pharmacother 2001, 2, 1479-1493), exogenous surfactants can be classified in four different types:
i) "natural" surfactants which are those recovered intact from lungs or amniotic fluid without extraction and have the lipid and protein composition of natural, endogenous, surfactant. They carry a potential infection risk because they cannot be sterilised, as heat denatures the hydrophilic proteins SP-A and SP-D. These surfactants are not available commercially;
ii) "modified natural" surfactants which are lipid extracts of minced mammalian lung or lung lavage. Due to the lipid extraction process used in the manufacture process, the hydrophilic proteins SP-A and SP-D are lost. These preparations have variable amounts of SP-B and SP-C and, depending on the method of extraction, may contain non-surfactant lipids, proteins or other components. Some of the modified natural surfactants present on the market, like Survanta (*vide ultra*) are spiked with synthetic components such as tripalmitin, dipalmitoylphosphatidylcholine and palmitic acid.
iii) "artificial" surfactants which are simply mixtures of synthetic compounds, primarily phospholipids and other lipids that are formulated to mimic the lipid composition and behaviour of natural surfactant. They are devoid of surfactant apoproteins;
iv) "reconstituted" surfactants which are artificial surfactants to which have been added surfactant proteins/peptides isolated from animals or proteins/peptides manufactured through recombinant technology such as those described in WO 95/32992, or synthetic surfactant protein analogues such as those described in WO 89/06657, WO 92/22315 and WO 00/47623.

### Modified natural surfactants on the market are:

- Surfactant TA, Surfacten^{®} available from Tokyo Tanabe, Japan
- Survanta^{®}, available from Abbott Laboratories, Illinois, USA
- Infasurf^{®}, available from Forrest Laboratories, Missouri, USA
- Alveofact^{®}, available from Thomae GmbH, Germany
- Curosurf^{®}, available from Chiesi Farmaceutici SpA, Italy

With these advances in surfactant therapy, neonatal deaths due to RDS and the various lung diseases related to surfactant deficiency no longer have the high rates they once had.

However, a significant percentages of cases fail to respond adequately to surfactant therapy. Numerous explanations for this lack of efficacy have been offered, the most likely ones invoking the inactivation of surfactant *in situ* by one or more substances that are normally absent from the alveolar spaces. The substances suspected of causing inactivation are those which have been implicated as the cause, or one of the contributing factors, of inactivation of endogenous surfactants in some of the diseases cited above, e.g. ARDS, and include blood proteins such as albumin, haemoglobin (Hb) and in particular fibrinogen and fibrin monomer, lipids and meconium (Fuchimukai T et *al J Appl Physiol* 1987, 62, 429-437; Cockshutt AM et al *Biochemistry* 1990, 36, 8424-8429; Holm BA et al *J Appl Physiol* 1987, 63, 1434-1442).

Resistance to inhibition therefore appears to be a desired characteristic for exogenous surfactant therapy in clinical disorders including both neonatal and adult RDS.

In some cases, inactivation of surfactants has been partly prevented by increasing the amount of administered surfactant. This is however not desirable because it is costly and also because the successful treatment of some diseases such as ARDS already involves the use of large and frequent doses. A further increase in the dose may thus negatively affect the clinical management of the patient.

Inhibition of surfactant has been thoroughly studied but the findings are not conclusive and the underlying mechanisms are not established.

According to Holm et al (*Chem Phys Lipids* 1990, 52:243-50) surfactants that are more sensitive to the inhibition by plasma proteins are the ones that lack surfactant proteins.

Hall et al (*Am Rev Respir Dis* 1992, 145, 24-30) found that the addition of SP-B and SP-C to an artificial surfactant devoid of proteins, Exosurf, increases to some degree the resistance to inactivation with albumin *in vitro* and *in vivo.*

Seeger et al. (*Eur Respir J* 1993, 6, 971-977) reported that various natural surfactant extracts and a reconstituted protein-containing synthetic surfactant mixture markedly differed in their sensitivity to inhibition by plasma proteins.

According to these authors, several aspects may underlie the marked differences in sensitivity to inhibition among the various surfactant preparations. Firstly, variations in lipid composition. Secondly, variations in protein composition. Modified natural surfactants which contain higher percentage of proteins, related to phospholipids, than reconstituted surfactants are normally more resistant to inhibition. Reconstituted surfactant containing recombinant SP-C and synthetic phospholipids showed a lower sensitivity to fibrinogen than that rid of the protein. Interestingly, however, this feature contrasted with a high sensitivity of such synthetic material towards the inhibitory capacity of haemoglobin, which suggests different underlying mechanisms of interference with surfactant function for fibrinogen and haemoglobin.

Thirdly, presence of contaminating materials. The presence of inhibiting substances derived from lung tissues could indeed explain the higher sensitivity to inhibition of modified natural surfactants obtained by extraction from minced lung tissues.

In another study (Walther et al *Respir Crit Care Med* 1997, 156, 855-861) it was found that supplementing Survanta with additional SP-B and SP-C significantly improved its function in the presence of, inactivating, plasma proteins.

Herting et al (Paediatric Research, 50 (1), 44-49, 2001) compared the inhibitory effects of human meconium on various surfactant preparations: Curosurf, Alveofact, Survanta, Exosurf, rabbit natural surfactant from bronchoalveolar lavage, and two reconstituted surfactants containing recombinant surfactant protein-C (rSP-C) or a leucine/lysine polypeptide (KL₄). Meconium is a complex mixture containing proteins, cell debris, bile acids, Hb, and bilirubin metabolites. All of these components are individually capable of inhibiting surfactant function. Aspiration of meconium can result in severe respiratory failure in term neonates. Surfactant inactivation is believed to play a key role in the pathophysiology of MAS (Meconium Aspiration Syndrome), and inhibition of the surface tension-lowering activity of surfactant by meconium has been demonstrated in vitro. In this study differences among modified natural surfactants Curosurf, Alveofact, or Survanta, which are currently in clinical use for treatment of neonatal RDS, were moderate. The reconstituted surfactants containing rSP-C and KL₄ were more resistant to inhibition than the modified natural surfactants. Natural surfactant containing SP-A was even more resistant to inactivation. The importance of SP-A in the resistance to surfactant inhibitors has also been demonstrated in previous studies. However, proteins such as SP-A, SP-B and SP-C are not readily available since they must either be isolated from natural surfactants or synthesised by recombinant or organic synthesis techniques.

Recently some authors have reported that the co-administration of some substances are able to reduce or prevent inactivation of surfactants.

In WO 00/10550, Taeusch et al have reported on the ability of non-ionic polymers and carbohydrates of reversing the inactivation of surfactants.

Dextrans, polyethylene glycols or polyvinylpyrrolidones in 1-10% (w/v), i.e. 10-100 mg/ml in the suspensions, were found to restore the ability of Survanta to lower the minimum surface tension in the presence of meconium, serum or lysophosphatidylcholine (Taeusch W et al *Pediatric Res* 1999, 45, 319A & 322A; Taeusch W et al *Am J Respir Crit Care Med* 1999, 159, 1391-1395).

The capability of 0.5-1.0% (w/v) dextran, i.e. 5-10 mg/ml in the suspensions, to restore the surface activity of albumin(serum)- or meconium-inhibited modified natural porcine surfactants has been reported (Konsaki T et al *Proceeding of the 35*^{*th*} *Scientific Meeting of Japanese Medical Society for Biological Interface,* October 9^{th}, 1999; Kobayashi et al. *J. Appl Physiol* 1999, 86, 1778-1784; Tashiro K et al *Acta Paediatr* 2000, 89, 1439-1445).

Tollofsrud et al *(Paediatric Res* 2000, 47, 378A) suggested that bovine serum albumin (BSA) can be used for blocking meconium free fatty acids (FFA), which in turn seem to be responsible for lung injury in MAS. BSA turned out to be effective in a ratio FFA/BSA of 1:1.

However, there are concerns on the use of hydrophilic polymers such as dextran as they could promote lung edema by increasing the colloid osmotic pressure in the airways. As far as BSA is concerned, it has been so far considered as one of the factor responsible of the inactivation of surfactants so its potential use for restoring their activity needs to be better investigated.

On the other hand, it might be possible to enhance the resistance of surfactant preparations by adding other components than naturally occurring, recombinant, or synthetic SPs. Accordingly, in view of the problems outlined above with respect to the components described in the prior art, the search for substances effective in counteracting the various form of surfactant inactivation continues.

### Disclosure of the invention

The present invention relates to a pharmaceutical composition as defined in claim 1.

Polymyxins are a family of antibiotics deriving from B. polymyxa (B. aerosporus).

In particular polymyxin B (PxB) is a highly charged amphiphilic cyclic peptidolipid of the formula sketched below, which has turned out to be useful in combating various fungal infections, especially those arising in immunocompromised individuals. 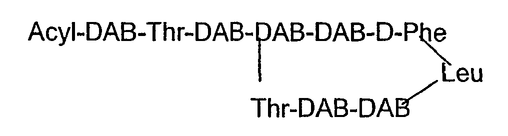 where:
DAB = L · α,γ-diaminobutyric acid
Polymyxin B is a mixture of polymyxin B₁ and polymyxin B₂
Thr is L-threonine
Phe is L-phenylalanine
Leu is L-leucine

The Acyl group is (+)-6-methyloctanoyl in polymyxin B₁ and 6-methyloctanoyl in polymyxin B₂.

The mechanism of action of polymyxin B, as antibiotic, in part relies upon the neutralization of endotoxin, accomplished by binding to the lipid A region of the endotoxin molecule. Endotoxins or lipopolysaccharides are structural components of the cell walls of the Gram-negative bacteria.

PxB has been reported to be able of cross-linking phospholipid vesicles by ionic interactions and promotes intervesicle lipid transfer (Cajal et al *Biochem Biophys Res Commun* 1995, 210, 746-752; Cajal et al *Biochemistry* 1996, 35, 5684-5695). This property was suggested to be involved in its antibacterial activity.

It has also been observed that PxB exhibits prolonged retention in the lung following pulmonary administration, which may be related to hydrophobic and electrostatic interactions between polypeptides and the phospholipids of lung (Mc Allister et al *Adv Drug Deliv Rev* 1996, 19, 89-110).

Zaltash et (*Biochim Biophys Acta* 2000, 146, 179-186), after having observed that the structural features of SP-B support a function in cross-linking of lipid membranes, found that a reconstituted surfactant spiked with PxB (which cross-links lipid vesicles but is structurally unrelated to SP-B) exhibited in vitro surface activity comparable to that of an analogous mixture containing SP-B instead of PxB. The reconstituted surfactant was made of an artificial mixture of phospholipids to which a peptidic analogue of SP-C was added.

WO 00/47623 claims particular peptidic analogs of SP-C, their use for preparing a reconstituted surfactant, useful in the treatment of respiratory distress syndrome (RDS), and other surfactant deficiencies or dysfunction as well as the use of polymyxins, in particular PxB as a substitute of SP-B.

Now it has been found, and it is the subject of the present invention, that polymyxins increase the resistance of pulmonary surfactants to inactivation as well as improve their surface properties.

In fact, it has been demonstrated that polymyxin B is able to restore the surface activity of albumin-inhibited Curosurf or reconstituted surfactant indicating that, by administering said additive, in combination with exogenous therapeutic pulmonary surfactants it is possible to reduce or fully counteract their inactivation.

In vitro experiments (pulsating bubble and micro bubble analysis) it has indeed been found that addition of PxB to 2.5 mg/ml Curosurf has a marked effect on the resistance to inhibition by albumin. In particular, it has been found that addition of 2% polymyxin relative to the surfactant mass, i.e. 0.05 mg/ml PxB in the solution, increases the resistance to inactivation of Curosurf by albumin.

It has also been found by pulsating bubble experiments that by addition of polymyxins, in particular polymyxin B, it is possible to improve the surface properties of Curosurf at low phospholipid concentrations.

Upon addition of such additive, the lowest concentration to which Curosurf could be diluted without losing its optimal properties in terms of minimum and maximum surface tension (γₘᵢₙ and γₘₐₓ), could be decreased about 2.5 times.

Furthermore, *in vivo* experiments carried out in immature newborn rabbits have proved that by addition of 2% polymyxin B, on the surfactant weight, to Curosurf, the surfactant dose could be significantly decreased without losing the beneficial effects of surfactant and that the incidence of pneumothorax during prolonged mechanical ventilation can be reduced as well.

As an extension of these findings, polymyxins can be useful for preparing compositions comprising modified natural surfactants or reconstituted/ artificial surfactants for clinical use in conditions wherein surfactant inhibition is expected. In particular, addition of polymyxins can allow to reduce required surfactant dose. For instance, in the case of ARDS, in which successful management according to the current clinical studies, requires large and frequent doses, it would be highly advantageous to reduce the dose of surfactant without affecting efficacy.

Possibly, natural modified surfactants or reconstituted/artificial surfactants fortified with polymyxins can also be useful for the treatment of BPD as it has been reported that these patients are subjected to a high incidence of pneumothorax.

So, as a further extension of these findings, natural modified surfactants or reconstituted/artificial surfactants fortified with polymyxins can also be particularly useful for the treatment of pulmonary syndromes, such as MAS, in which excessive or highly concentrated meconium or other secretions poses a threat to the health and safety of foetuses and newborns.

In general terms, a composition containing as active ingredients a pulmonary surfactant that is effective for the treatment of pulmonary disorders related to a lack and/or dysfunction of endogenous surfactant in combination with a polymyxin can be an effective therapeutic agent for the treatment of several pulmonary disorders, with an effect that is expected to be greater than that achieved with administration of surfactant alone.

Examples of such disorders include neonatal and adult respiratory distress syndromes, meconium aspiration syndrome, any type of pneumonia and possibly bronchopulmonary dysplasia.

Therefore, the invention also refers to said compositions as a novel mean for treating, reducing or preventing the aforementioned disorders.

### Detailed disclosure of the invention

The additives utilised in the present invention are polymyxins, a family of five antibiotics A, B, C, D, E and any salt thereof. Useful polymyxins include those deriving from B. polymyxa (B. aerosporus) as well as those that can be synthesised in the laboratory. A preferred polymyxin is polymyxin B, more preferably as sulphate.

The pulmonary surfactants used in the practice of the present invention are a modified natural pulmonary surfactant or an artificial surfactant as defined in claim 1. They are effective in the treatment of pulmonary disorders. The surfactants include biological substances that are obtained from animal sources, preferably mammalian lungs, and then treated by supplementation, extraction or purification. Preferably the surfactant is a modified natural surfactant selected from the group of Surfactant TA, Survanta, Infasurf, Alveofact. The preferred surfactant is Curosurf.

Other surfactants which can be used are artificial surfactants sensitive to the inhibition by plasma proteins or by other inhibiting agents.

The proportion of polymyxin relative to the surfactant can vary. In general best results are achieved with a percentage of polymyxin expressed as sulphate between 0.1 and 10% on the weight of the surfactant (w/w), preferably between 0.5 and 5%, most preferably between 1 and 3%: the man skilled in the art will identify each time the suitable percentage.

The additive can be administered either before, during or after the administration of the surfactant and, in any case, both are administered directly or indirectly to the lungs of the patients.

In a particularly convenient method, the polymyxin and the surfactant are combined in a single aqueous liquid formulation which is then administered to the patient. In an even more convenient method, the polymyxin and the surfactant are administered as a suspension in buffered physiological saline.

Suitable methods for administration are the same as those generally considered suitable and effective for surfactant therapy. The most direct and effective method is instillation of the composition into the lungs through the trachea. Another suitable method of administration is in form of aerosol, in particular by nebulization. The amounts of the components will be based on the surfactant dosage in accordance with the dosages currently used for surfactant therapy. Modified natural surfactants are typically supplied as suspension in single-use glass vials. The surfactant concentration (expressed as phospholipid content) is in the range of from about 2 to about 160 mg of surfactant per ml, preferably between 10 and 100 mg/ml, more preferably between 20 and 80 mg/ml.

According to the teaching of the present invention, polymyxins as additives are useful to supplement and improve surfactant therapy for a variety of pulmonary disorders, abnormal conditions and diseases caused or related to a deficiency or dysfunction of the pulmonary surfactant. Examples are hyaline membrane disease, neonatal and adult respiratory distress syndromes, acute lung injury (such as that resulting from ozone inhalation, smoke inhalation or near drawing), conditions of surfactant inactivation triggered by volutrauma and barotrauma, meconium aspiration syndrome, capillary leak syndrome, bacterial and viral pneumonia, and bronchopulmonary dysplasia.

The following examples illustrate in detail the invention.

### Experimental part

### Preparation of surfactant

Curosurf (80 mg/ml phospholipid fraction from porcine lung, equivalent to about 74 mg/ml of total phospholipids and about 1 mg of protein) was diluted in 0.9% NaCl to phospholipid concentrations of 0.1, 1, 1.5, 2, 2.5, 3.5 and 5 mg/ml. To some preparations 1%, 2% or 3% of polymyxin B (Polymyxin B Sulfate, Sigma) relative to the amount of surfactant phospholipids was added.

### Exposure to surfactant inhibitor

Resistance to albumin inhibition was tested by addition of human serum albumin (Human albumin, Sigma), 0.1, 0.4, 0.5, 1.6, 2.5, 10 or 40 mg/ml to the different surfactant preparations. All surfactant preparations were kept at 37°C for 1 hour prior to the surface tension measurements.

### Surface tension measurements

Surface properties were determined in a pulsating bubble system (Surfactometer International, Toronto, Canada). The plastic chamber was filled with the test fluid (approximately 20 µl) and a bubble was created. The bubble was kept at static conditions for 1 min and then pulsated with 50% cyclic compression of the bubble surface for 5 min at 37°C at a rate of 40 pulses per minute. Pressure across the bubble wall was recorded during the 5^{th} cycle, and after 1, 2 and 5 min of pulsation. Values for surface tension at maximum and minimum bubble diameter were calculated according to the LaPlace equation: P=2γ/r, where P is the measured pressure gradient across the bubble wall, r the radius, and γ the surface tension. Values were obtained from 5-7 observations for each sample.

### Microbubble analysis

Surfactant preparations were analysed at 0.1 mg/ml after being shaken vigorously for 30 sec, and the number and percentage of microbubbles (diameter <20 µm) generated in the suspension was determined by computerized image analysis essentially as described by Berggren et al (*Biol Neonate,* 1992, 61 (suppl 1), 15-20).

### Spreading experiments

Experiments were performed at 37°C using a modified Wilhelmy balance with a surface area of 20 cm². Standard amounts of Curosurf at various concentrations (10-80 mg/ml) were applied onto a hypophase of saline, approximately 40 mm from the dipping plate. In some experiments, CaCl₂ (2 mM), dextran (30 mg/ml), or polymyxin B (2% on the weight of the surfactant, i.e. 0.4 mg/ml) was added to diluted surfactant material (20 mg/ml). The effect of albumin at low concentration (0.1 mg/ml) added to the hypophase was also investigated. Surface tension, measured 1 sec after administration of the sample was used as a parameter combining spreading and film formation by adsorption fom the hypophase.

### Statistics

Data were expressed as mean ± SD. The CRISP statistical program (Crunch Software, San Francisco CA) was used for data analysis. Differences were evaluated by one-way analysis of variance (ANOVA) followed by Student-Newman-Keuls test. A P-value ≤ 0.05 was regarded as statistically significant.

### Example 1 - Effect of polymyxin B on the surfactant resistance to inhibition by albumin at different concentrations

The effect of PxB on Curosurf against inhibition by albumin at different concentrations was evaluated by using the pulsating bubble surfactometer (PBS) and by analysis of microbubble stability.

### Pulsating bubble surfactometer (PBS) experiments

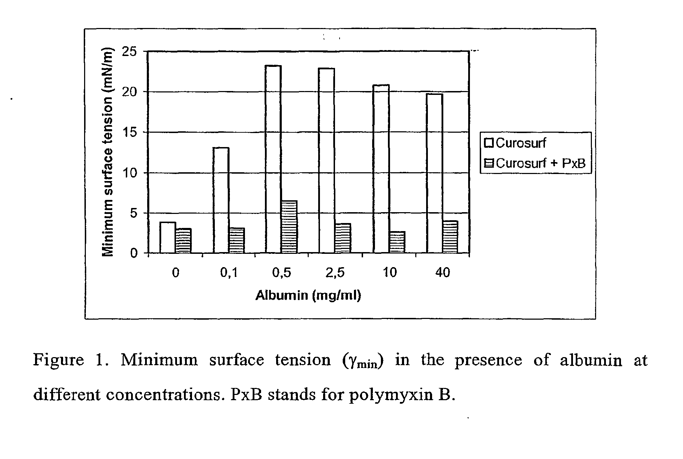

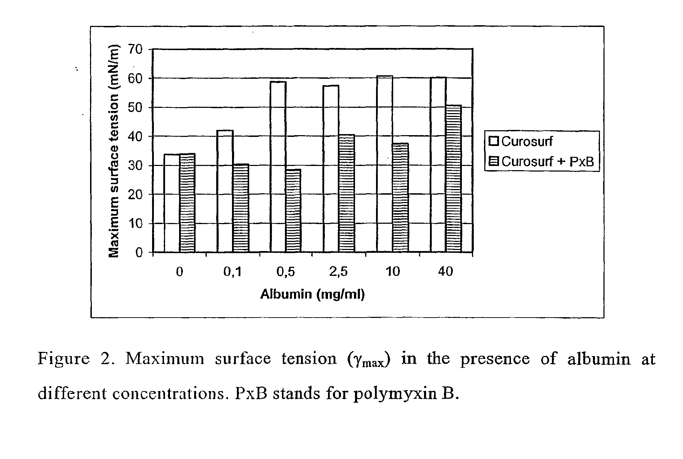

For the PBS experiments albumin at concentrations up to 40 mg/ml were added to Curosurf at 2.5 mg/ml, or Curosurf at 2.5 mg/ml containing 2% (w/w) of PxB, and the surface tension at minimum and maximum bubble radius (γₘᵢₙ, γₘᵢₙ) after 5 min of pulsation at 37°C and 40 cycles/min were recorded. All experiments were performed in triplicate and the mean values are shown. Figure 1 illustrates that γₘᵢₙ remains <5 mN/m for the PxB-containing preparation also in the presence of 40 mg/ml albumin, while without PxB γₘᵢₙ increases dramatically already at albumin concentrations ≥0.1 mg/ml. Likewise, in the absence of PxB the γₘₐₓ values increase significantly at albumin concentrations >0.1 mg/ml, while in the presence of PxB γₘₐₓ remains <40 mN/m up to 10 mg/ml of albumin (Figure 2).

### Microbubble stability experiments

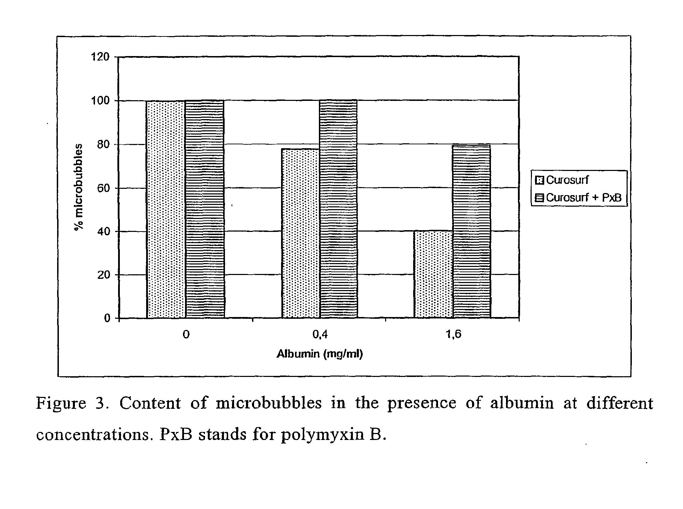

Microbubble stability analysis also shows a prominent effect of PxB. Figure 3 shows that in the presence of PxB the contents of microbubbles remain high up to 1.6 mg/ml of albumin. Without PxB, addition of albumin causes a dose-dependent decrease in the percentage of microbubbles, and in the presence of 1.6 mg/ml albumin the surfactant preparation contains only 40% microbubbles.

The conclusion from both the PBS and the microbubble analysis is that addition of PxB to 2.5 mg/ml Curosurf has a marked effect on the resistance to inhibition by albumin.

### Example 2 - Effects of polymyxin B on Curosurf

### Effects of polymyxin B on Curosurf and its sensitivity to albumin 40 mg/ml

Different concentrations of Curosurf and Curosurf plus 2% polymyxin B were tested in order to find the lowest concentration at which the surfactant preparations possess optimal surface properties without added albumin or in the presence of albumin. Optimal surface properties were defined as minimum surface tension (γₘᵢₙ) <5 mN/m and maximum surface tension (γₘₐₓ) <35 mN/m after 5 min of pulsation in the pulsating bubble surfactometer. The surfactant concentrations were then stepwise diluted until γₘᵢₙ was > 15 mN/m and γₘₐₓ >50 mN/m.

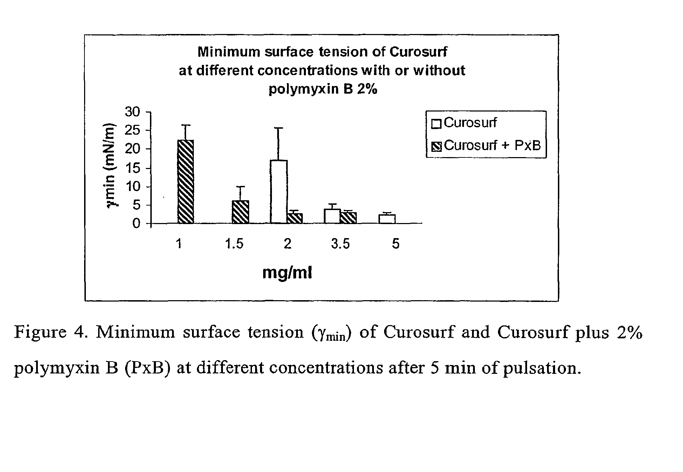

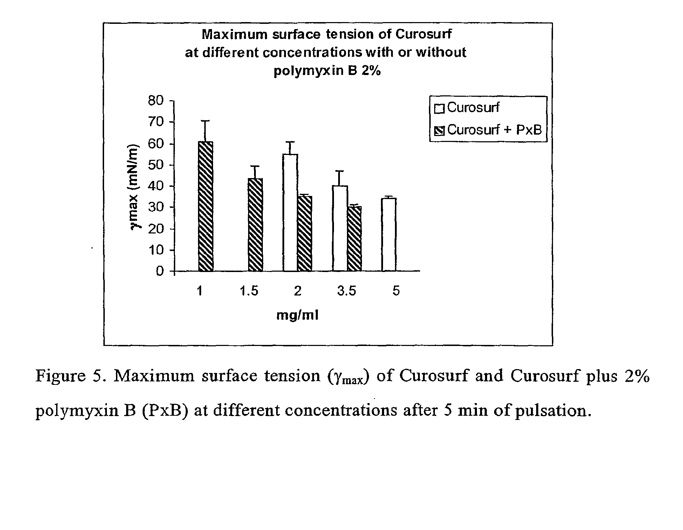

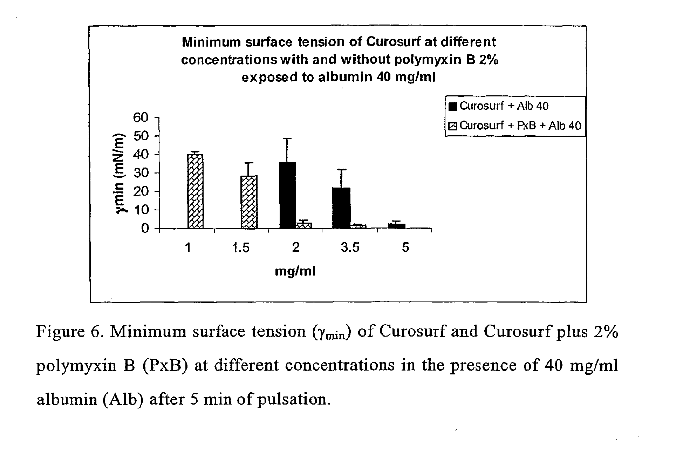

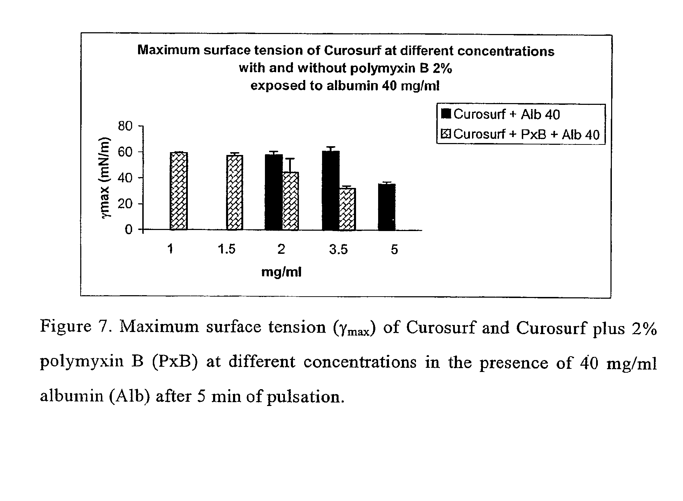

As seen in Figs. 4-7 optimal surface properties were recorded at a concentration of 5 mg/ml of Curosurf, with a γₘᵢₙ of 2.2 ± 0.7 mN/m and γₘₐₓ of 33.9 ± 1.1 mN/m. Curosurf at this concentration was resistant to albumin, 40 mg/ml. With lower concentrations both γₘᵢₙ and γₘₐₓ increased, especially in the presence of albumin.

Addition of 2% polymyxin B improved surface properties of Curosurf at low phospholipid concentrations. When 2% polymyxin B was added to Curosurf, 2mg/ml, γₘᵢₙ decreased from 16.8 ± 8.9 to 2.7 ± 0.8 mN/m (P<0.05) (Fig. 4) and γₘₐₓ from 54.8 ± 5.9 to 35.0 ± 1.0 mN/m (P<0.01) (Fig. 5). In the presence of 40 mg/ml of albumin addition of 2% polymyxin B led to a decrease of γₘᵢₙ from 35.7 ± 13.2 to 3.0 ± 1.2 mN/m (P<0.01) (Fig. 6) and γₘₐₓ from 57.9 ± 2.8 to 44.5 ± 10.7 mN/m (P<0.05) (Fig. 7).

Addition of 2% polymyxin B thus improved the surface properties of Curosurf at low phospholipid concentrations and increased the resistance to inactivation of Curosurf with albumin. The results showed that the lowest concentration of Curosurf with optimal in vitro properties could be decreased about 2.5 times by addition of 2% polymyxin B.

### Example 3 - Effects of different concentrations of polymyxin B in Curosurf

Pulsating bubble experiments were employed for evaluating the optimal concentration of polymyxin B to maintain optimal surface activity of 2 mg/ml concentration of Curosurf either without added albumin or in presence of albumin 40 mg/ml. The results indicate that the optimal concentration range of polymyxin B is comprised between 1% and 3% on the weight of surfactant and 2% is the preferred one.

### Example 4 - Spreading experiments

With high concentration/low volume samples (80 mg/ml; 10 microl), spreading was very effective and mean value for surface tension at 1 sec (28 mN/m) was only slightly higher than equilibrium surface tension (24 mN/m) established within 10 sec. With low concentration/large volume samples containing the same amount of Curosurf (10 mg/ml; 80 microl), spreading was delayed and characteristically biphasic, mean value for surface tension at 1 sec significantly higher (60 mN/m) and equilibrium surface tension < 25 mN/n was not reached until after about 30 sec. Surface spreading of diluted samples (20 mg/ml) was further retarded by addition of albumin to the hypophase, but accelerated by high concentration of dextran or by low amount of polymyxin B, also in the presence of albumin. Addition of CaCl₂ had no such effects (Table 1).

**Table 1 - Spreading rates of Curosurf diluted to 20 mg/ml (applied volume 10 microl), after addition of various agents to the surfactant material or to the hypophase; mean values from a minimum of 3 measurements.**

| Material added | Surface tension at 1 sec (mN/m) |
|---|---|
| - | 52 |
| CaCl₂ (2 mM) | 52 |
| Dextran (30 mg/ml) | 33 |
| Polymyxin B (2%, = 0.4 mg/ml) | 28 |
| Albumin (0.1 mg/ml)* | 70 |
| Albumin* + CaCl₂ | 70 |
| Albumin* + dextran | 39 |
| Albumin* + polymyxin B | 45 |

| | |
|---|---|
| *Albumin was added to the hypophase | |

The data show that spreading of diluted surfactant samples can be enhanced in vitro by addition of 0.4 mg/ml PxB (2% w/w), i.e. at low concentrations compared to total surfactant phospholipids, which is significantly lower than the concentration of dextran (30 mg/ml equivalent to 150% w/w) required to obtain a similar effect.

### Example 5 - In vivo experiments

The animal experiments were designed to test the hypothesis that by addition of 2% polymyxin B to Curosurf the surfactant dose could be decreased from 200 to 80 mg/ kg body weight, i.e. 2.5 times, without losing the beneficial effects.

Preterm rabbit fetuses (New Zealand White) were delivered at a gestational age of 27 days by caesarean section (term=31 days). At delivery, the animals were anaesthetized with intraperitoneal sodium pentobarbital (0.1 ml; 6 mg/ml), tracheotomized, paralyzed with intraperitoneal pancuronium bromide (0.1-0.15 ml; 0.2 mg/ml) and kept in plethysmograph system at 37°C. They were mechanically ventilated in parallel with a modified Servo-Ventilator (900B, Siemens-Elema, Solna, Sweden) delivering 100% oxygen. The working pressure was set at 55 cmH₂O. The frequency was 40 per minute, the inspiration:expiration time ratio 1:1 and tidal volume was adjusted between 8-10 ml/kg body weight. No positive expiratory pressure was applied.

The immature newborn rabbits were randomized to receive at birth, via the tracheal cannula, 2.5 ml/kg body weight of Curosurf (32 or 80 mg/ml) or 2% polymyxin B in Curosurf (32 mg/ml). In control animals, no material was instilled into the airways. All animals were ventilated for 5 hours.

Electrocardiograms (ECG) were recorded by means of subcutaneous electrodes. The ECG was checked at the same intervals as indicated above. As soon as arrhythmia, bradycardia (heart rate < 60/min), absence of QRS complexes or pneumothorax occurred, animals were sacrificed by intracranial injection of lidocaine (0.5 ml; 20 mg/ml). All animals surviving 5 hours were killed with the same method. The time of survival in all animals was recorded. The abdomen was opened and the diaphragm inspected for evidence of pneumothorax.

### Lung function measurements

The peak inspiratory pressure (PIP) was recorded with a pressure transducer (EMT 34) and individually adjusted for each animal to obtain tidal volume (V_{T}) 8-10 ml/kg body weight. Tidal volume was recorded with Fleisch-tube, a differential pressure transducer (EMT 31), an integrator (EMT 32), an amplifier (EMT 41) and a recording system (Mingograf 81; all equipment, Siemens-Elema). The system was calibrated for each individual experiment and a linear calibration curve was obtained for tidal volumes between 0.1 and 0.8 ml. Lung-thorax compliance was derived from recordings of tidal volume and peak inspiratory pressure and expressed in ml/kg.cmH₂O.

**Table 2 - Number of animals (n), body weight, incidence of pneumothorax and number of survivors at 180 min.**

| **Group** | **n** | **Body weight (g)** | **Pneumothorax (n)** | **Survivors at 180 min (n)** |
|---|---|---|---|---|
| Control | 17 | 29.2±4.4 | 10 | 0 |
| Curosurf 32 mg/ml + polymyxin B 2% | 17 | 28.3±3.6 | 2 | 7 |
| Curosurf 32 mg/ml | 18 | 29.1±3.5 | 8 | 2 |
| Curosurf 80 mg/ml | 16 | 29.9±4.4 | 5 | 6 |

| | | | | |
|---|---|---|---|---|
| Values are presented as means ± SD. | | | | |

All three groups of surfactant-treated animals had higher compliance values than controls at 15 min. These experiments prove that by addition of 2% polymyxin B to Curosurf, the surfactant dose could be significantly decreased without losing the beneficial effects of surfactant, and the incidence of pneumothorax during prolonged mechanical ventilation could be reduced.

## Claims

1. A pharmaceutical composition comprising:
(a) a surfactant effective in the treatment of pulmonary disorders, abnormal conditions and diseases due to a lack or deficiency of endogenous surfactant;
(b) a polymyxin
wherein the surfactant is a modified natural pulmonary surfactant consisting of a lipid extract of minced mammalian lung or lung lavage containing the surfactant protein SP-B and SP-C or an artificial surfactant consisting of a mixture of synthetic compounds, primarily phospholipids and other lipids that are formulated to mimic the lipid composition and behaviour of natural surfactant and that are devoid of surfactant apoproteins.

2. A composition according to claim 1 wherein said surfactant is a modified natural surfactant (**for example,** surfactant TA, Survanta^{®}, Infasurf^{®}, Alveofact^{®} or Curosurf^{®}).

3. A composition according to claim 1 wherein said surfactant is an artificial surfactant.

4. A composition according to claims 1-3, wherein said polymyxin is polymyxin B.

5. A composition according to the preceding claims wherein the percentage of said polymyxin is comprised between 0.1 and 10% on the weight of the surfactant.

6. A composition according to claim 5 wherein the percentage is comprised between 0.5 and 5%.

7. A composition according to claim 5 wherein the percentage is comprised between 1 and 3%.

8. A composition according to claims 1-7 in the form of aqueous suspension.

9. A pharmaceutical composition according to claim 8 comprising a modified natural pulmonary surfactant in a concentration comprised between 2 and 160 mg/ml.

10. A composition according to claim 9 wherein the concentration of modified natural pulmonary surfactant is comprised between 20 and 80 mg/ml.

11. A composition according to any one of claims 1-10 wherein said pulmonary disorders, abnormal conditions and diseases include neonatal and adult respiratory distress syndromes, meconium aspiration syndrome, bacterial and viral pneumonia, bronchopulmonary dysplasia.

## Patentansprüche

1. Pharmazeutische Zusammensetzung, umfassend:
(a) ein Surfactant, das wirksam ist bei der Behandlung von Lungenerkrankungen, abnormalen Zuständen und Krankheiten aufgrund eines Mangels oder einer Defizienz an endogenem Surfactant;
(b) ein Polymyxin
wobei das Surfactant ein modifiziertes natürliches Lungensurfactant, bestehend aus einem Lipidextrakt aus zerkleinerter Säugerlunge oder Lungenspülung, enthaltend die Surfactant-Proteine SP-B und SP-C, oder ein künstliches Surfactant, bestehend aus einem Gemisch synthetischer Verbindungen, vorrangig Phospholipide und andere Lipide, die formuliert sind, um die Lipidzusammensetzung und das Verhalten von natürlichem Surfactant zu imitieren, und die frei von Surfactant-Apoproteinen sind, ist.

2. Zusammensetzung nach Anspruch 1, wobei das Surfactant ein modifiziertes natürliches Surfactant (zum Beispiel Surfactant TA, Survanta^{®}, Infarsurf^{®}, Alveofact^{®} oder Curosurf^{®}) ist.

3. Zusammensetzung nach Anspruch 1, wobei das Surfactant ein künstliches Surfactant ist.

4. Zusammensetzung nach den Ansprüchen 1-3, wobei das Polymyxin Polymyxin B ist.

5. Zusammensetzung nach den vorstehenden Ansprüchen, wobei der prozentuale Anteil an Polymyxin zwischen 0,1 und 10 Prozent, bezogen auf das Gewicht des Surfactants, liegt.

6. Zusammensetzung nach Anspruch 5, wobei der prozentuale Anteil zwischen 0,5 und 5 Prozent liegt.

7. Zusammensetzung nach Anspruch 5, wobei der prozentuale Anteil zwischen 1 und 3 Prozent liegt.

8. Zusammensetzung nach den Ansprüchen 1-7 in der Form einer wässrigen Suspension.

9. Pharmazeutische Zusammensetzung nach Anspruch 8, umfassend ein modifiziertes natürliches Lungensurfactant in einer Konzentration zwischen 2 und 160 mg/ml.

10. Zusammensetzung nach Anspruch 9, wobei die Konzentration an modifiziertem natürlichem Lungensurfactant zwischen 20 und 80 mg/ml liegt.

11. Zusammensetzung nach einem der Ansprüche 1-10, wobei die Lungenerkrankungen, abnormalen Zustände und Krankheiten Atemnotsyndrome von Neugeborenen und Erwachsenen, Meconium-Aspirations-Syndrom, bakterielle und virale Pneumonie, bronchopulmonale Dysplasie umfassen.

## Revendications

1. Composition pharmaceutique comprenant :
(a) un agent tensioactif efficace dans le traitement de troubles pulmonaires, d'affections anormales et de maladies dus à un manque ou une déficience d'agent tensioactif endogène ;
(b) une polymyxine
dans laquelle l'agent tensioactif est un agent tensioactif pulmonaire naturel modifié consistant en un extrait lipidique de poumon mammifère émincé ou de lavage pulmonaire contenant la protéine d'agent tensioactif SP-B et SP-C ou un agent tensioactif artificiel consistant en un mélange de composés synthétiques, principalement des phospholipides et d'autres lipides qui sont formulés pour imiter la composition lipidique et le comportement d'un agent tensioactif naturel et qui sont dépourvus d'apoprotéines d'agent tensioactif.

2. Composition selon la revendication 1, dans laquelle ledit agent tensioactif est un agent tensioactif naturel modifié (par exemple, agent tensioactif TA, Survanta®, Infasurf®, Alveofact® ou Curosurf®).

3. Composition selon la revendication 1, dans laquelle ledit agent tensioactif est un agent tensioactif artificiel.

4. Composition selon les revendications 1 à 3, dans laquelle ladite polymyxine est la polymyxine B.

5. Composition selon les revendications précédentes, dans laquelle le pourcentage de ladite polymyxine est compris entre 0,1 et 10 % en poids de l'agent tensioactif.

6. Composition selon la revendication 5, dans laquelle le pourcentage est compris entre 0,5 et 5 %.

7. Composition selon la revendication 5, dans laquelle le pourcentage est compris entre 1 et 3 %.

8. Composition selon les revendications 1 à 7, sous la forme d'une suspension aqueuse.

9. Composition pharmaceutique selon la revendication 8, comprenant un agent tensioactif pulmonaire naturel modifié en une concentration comprise entre 2 et 160 mg/ml.

10. Composition selon la revendication 9, dans laquelle la concentration d'agent tensioactif pulmonaire naturel modifié est comprise entre 20 et 80 mg/ml.

11. Composition selon l'une quelconque des revendications 1 à 10, dans laquelle lesdits troubles pulmonaires, affections anormales et maladies incluent les syndromes de détresse respiratoire du nouveau-né et de l'adulte, le syndrome d'aspiration du méconium, une pneumonie bactérienne et virale, une dysplasie bronchopulmonaire.
